(19)

**Europäisches Patentamt**
**European Patent Office**
**Office européen des brevets**

(11) **EP 4 748 920 A1**

(12) # EUROPEAN PATENT APPLICATION

(43) Date of publication:
**27.05.2026 Bulletin 2026/22**

(21) Application number: **25215784.7**

(22) Date of filing: **14.11.2025**

(51) International Patent Classification (IPC):
*C12M 1/24* (2006.01)     *A01N 1/147* (2025.01)
*A61K 35/00* (2006.01)    *B01F 31/22* (2022.01)
*B01L 7/00* (2006.01)     *C12M 3/06* (2006.01)
*C12M 1/00* (2006.01)     *A01N 1/125* (2025.01)
*A01N 1/142* (2025.01)

(52) Cooperative Patent Classification (CPC):
**A01N 1/147; A01N 1/142;** C12M 23/08

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.11.2024 EP 24214697
28.11.2024 EP 24216155
20.12.2024 EP 24222065**

(71) Applicant: **Bayer Healthcare LLC
Whippany, NJ 07981 (US)**

(72) Inventors:
• **Rekic, Nikos
51373 Leverkusen (DE)**

• **Lönner, Maren
51373 Leverkusen (DE)**
• **Maiser, Benjamin
51373 Leverkusen (DE)**
• **Svenja, Jorga
51373 Leverkusen (DE)**
• **Schmidt, Wolfgang
51373 Leverkusen (DE)**
• **Ritter, Joachim
51373 Leverkusen (DE)**

(74) Representative: **BIP Patents
c/o Bayer Intellectual Property GmbH
Alfred-Nobel-Straße 50
40789 Monheim am Rhein (DE)**

(54) **AUTOMATED ASEPTIC FORMULATION METHOD**

(57)    What is described herein relates to automated aseptic method for formulation of cells comprising providing a container with a filling volume in the range of between of 0,01-50 ml, filling the container with a cell suspension and a solution, closing the container, mixing the cell suspension and the solution to generate a final suspension, wherein the container is a final product container

EP 4 748 920 A1

**Description**

BACKGROUND

Cell therapy products require different processes from methods where the cells are merely the production site of a product e.g. an antibody. For cell therapy products it is crucial to ensure process traceability, maintain consistent and reproducible conditions and process times, and minimize user-dependent variability, such as that caused by manual transfers between process steps.

Hence there is a need for a fully aligned process that standardizes individual steps and minimizes manual variability through process design and a higher degree of automation.

BRIEF DESCRIPTION OF THE DRAWINGS

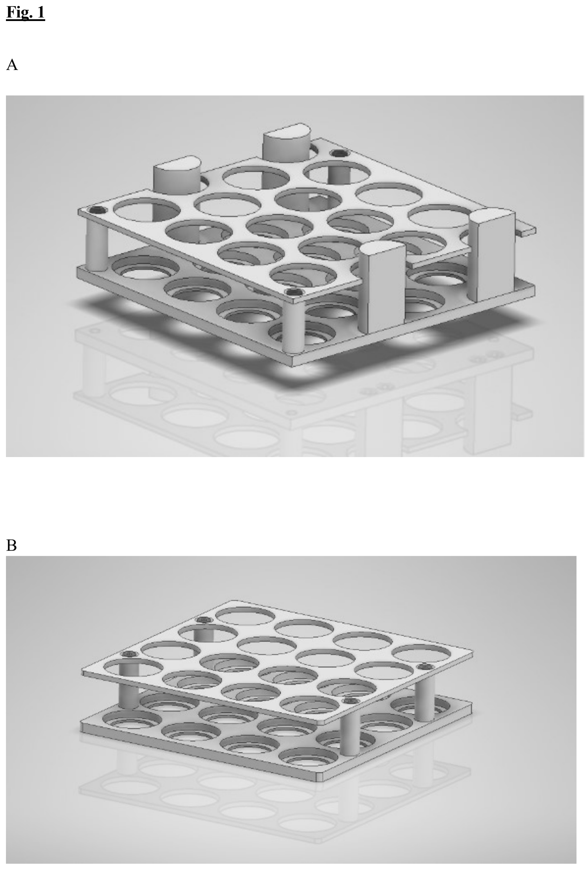

Fig 1A and 1B show computer aided design (CAD) exemplary models of racks, created with Autodesk Inventor Pro, also termed cryo-nests.

The rack shown in Fig 1A has a width and length of 115,5 mm with a height of 34 mm. The rack of Fig 1A has an alternating arrangement of the vials, including half dummy vials to keep the distances between the vials constant. It holds 14 product vials which according to CFD simulations all experience the same flow of cooling gas during freezing hence ensuring a consistent product quality.

The rack shown in Fig 1B has a width and length of 115,5 mm and a height of 26 mm. It has a checkerboard pattern arrangement of the vials, which makes better use of the space than the rack of Fig 1A and thus allows a higher loading of 16 vials.

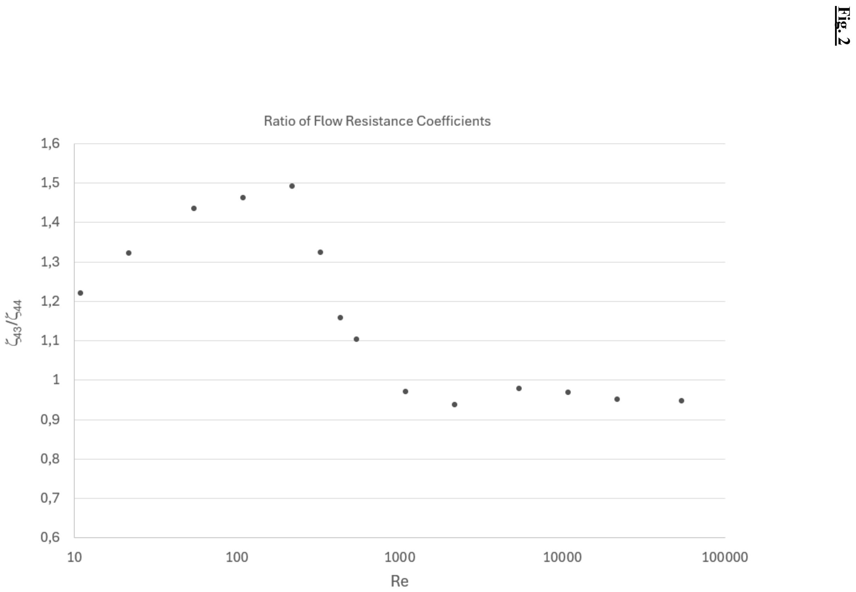

Fig. 2 shows ratio of flow resistance coefficients over Reynolds number of completely filled racks as shown in Fig. 1A and Fig. 1B.

DESCRIPTION

What is described herein in a first aspect relates to an improved method for cell product formulation. In detail what is described herein in a first aspect relates to an automated aseptic method for formulation of cells comprising

- providing a container with a filling volume in the range of 0,01-50 ml
- filling the container with a cell suspension and a solution
- closing the container
- mixing the cell suspension and the solution to generate a final suspension.

In a preferred embodiment the method of the first aspect relates to an automated aseptic method for formulation of cells comprising

- providing a container with a filling volume in the range of 0,01-50 ml
- filling the container with a cell suspension and a solution
- closing the container
- mixing the cell suspension and the solution to generate a final suspension

wherein the container is the final product container.

Currently formulation of cells e.g. for cryopreservation is typically performed batch-wise by adding the cryoprotectant to a cell pellet or as a concentrate to a cell suspension followed by thorough mixing. Afterwards the cell suspension is filled into (cryo)vials or cryobags and is subsequently subjected to container closure integrity testing, visual inspection, labeling, and a final batch-wise freezing. The cell therapy products that undergo cryopreservation require a formulation that supports cell viability during freezing, storage and thawing processes. DMSO is the commonly used cryoprotectant. While DMSO is crucial for cell survival during cryopreservation, it is harmful to cells when not frozen (cf. Example 6). This necessitates minimizing the exposure time of cells to DMSO prior to and after freezing.

The method of the first aspect is hence advantageous as minimizes the exposure time of any given cell to DMSO prior to freezing.

In addition, since each vial and thereby all cells undergoing formulation and subsequent mixing, experience the same controlled conditions throughout the method of the first aspect, this method is advantageous as it delivers a consistent product quality via ensuring a narrow residence time distribution for any given vial.

Moreover, the step of filling the final solution into the final product container after mixing the cells and the cryopreservation is omitted, rendering the novel method more time and cost efficient.

As used herein the term "cell suspension" refers to single cells or small cell agglomerates of 1-100 cells in a buffered aqueous solution.

As used herein the term "automated" refers to formulation process of cell products with minimal or no manual handling.

As used herein the term "aseptic processing" refers to prevention of contamination of the cell suspension with viable or non viable contaminates from the environment e.g. via to sterile handling. This is important as cells cannot be sterilized as biomolecules could be. Hence, materials, containers, devices and the product environment are controlled to prevent microbial, endotoxin/pyrogen and particle contamination (EudraLex, Volume 4, Annex 1).

As used herein, the term "formulation" refers to a suspension of cells and solution consisting of specified ingredients in predetermined amounts or proportions. Therefore, in the context of the method of the first aspect formulation refers to the fact that the (formulated) final suspension comprises cells and solution in a predetermined amount and proportion and that it is not merely a combination but that the cells and the solution form a suspension i.e. have been mixed.

As mentioned above in one embodiment of the method of the first aspect the container is the final product container.

As used herein the term "final product container" refers to a container e.g. a vial, in which the cells are stored i.e. from which they are not removed again for a time in the range of 10 hours to 48 months, preferably 24 hours to 12 months, even more preferably 48 hours to 6 months.

Hence, in a preferred embodiment of the method of the first aspect the method relates to an automated aseptic method for formulation of cells comprising

- providing a container with a filling volume in the range of 0,01-50 ml
- filling the container with a cell suspension and a solution
- closing the container
- mixing the cell suspension and the solution to generate a final suspension

wherein the container is the final product container and
wherein the final suspension is not removed again from the final product container for a time in the range of 10 hours to 48 months, preferably 24 hours to 12 months, even more preferably 48 hours to 6 months.

In a further embodiment of the method of the first aspect the method comprises the additional step of cryopreserving the cells in the final product container.

Preferably the final product container is also the container in which the cells are frozen, thereby omitting a filling step after mixing.

Hence, in a preferred embodiment of the method of the first aspect the final product container is the container in which the cells are frozen, thereby omitting a filling step after mixing.

The cells can be selected from eukaryotic or prokaryotic cells. Eukaryotic cells can be selected from animal or plant cells. Preferably the eukaryotic cells are pluripotent cells such as human embryonic stem cells or human iPS cells or cell differentiated from these cell types.

An exemplary way of generating enough cells that could to be used e.g. in the method described herein can be summarized as follows:

Pluripotent stem cells - e.g. hES cells or hiPS cells - are seeded at 6.000 - 20.000 cells/cm2 in a suitable medium such as mTeSR-plus or StemScale into an adherent cell culture vessel. In case an adherent cell culture vessel such as a T-flask or a cell factory is employed, anti-apoptotic reagents such as a combination of Chroman 1, Emricasan, Polyamine and Trans-ISRIB (CEPT) should be added. Moreover, the adherent cell culture vessel is either precoated with laminin or 0.2 - 0.3 $\mu$g/cm$^2$ laminin-511 E8 fragment protein e.g. LN511-E8 are directly added with the cells to the vessel. Complete medium is exchanged every day or every other day with the addition of supplements. After 4 days the cells are harvested using Accutase or Versene and again seeded at 6.000 - 20.000 cells/cm2 in the complete medium supplemented with either Ri or CEPT reagent into a precoated cell culture vessel or with direct addition of 0.2 - 0.3 $\mu$g/ cm2 laminin fragment e.g. LN511-E8. Thereafter the complete medium without CEPT is replaced every day or every other day. At day 8 the cells are again harvested - optionally via a clump passaging method - using Accutase and seeded into a non-adherent cell culture vessel at 0.10-0.4 million cells/ml in the same medium used before with supplemented with a combination of Chroman 1, Emricasan, Polyamine and Trans-ISRIB and optional addition of 0.1% Pluronic. For further cell cultivation the glucose concentration is maintained at 1.5g/L-4g/L, the pH is maintained at 7 and optionally the Na2CO3 is maintained at 7.5%. Thereafter medium and if applicable the optional supplements are exchanged at rate of 50% to 200% vessel volume every day or according to the cell specific perfusion rate. The cells are harvested between day 15 and day 17 using Accutase or Accumax and then used as described herein. Alternatively, the cells are frozen as batch or differentiated. As stated above instead of culturing the cells in an adherent manner first, the cells can also be directly seeded into a non-adherent cell culture vessel at 0.10-0.4 million cells/ml.

Preferably the container is a vial. Preferably the vial is made from COC (Cyclic Olefin-Copolymer) or COP (Cyclic Olefin Polymer).

Preferably the vial has a filling volume in the range of 0,1 ml - 25ml, even more preferably in the range of 1-5 ml, most preferred of 2ml.

In an embodiment of the method of the first aspect the container is closed with a stopper. Preferably the stopper is made from Bromobutyl or Butyl (Flurotec®) optional with a coating like FluroTec(R). Moreover, closing of the container occurs automatically via a crimping device (e.g. disc crimping or jaw crimping) such as the disc crimper model 4610 from Bausch and Ströbel or the jaw crimper from Thermo Fisher (catalog number 60180-ECRH13KI).

In one embodiment the cell suspension is added to the container first and the solution is added after the cell suspension to the container.

This embodiment has the advantage that the post thaw viability, frequency of apoptotic cells and replating efficiency were comparable to manually handled cells.

In an alternative embodiment the solution is added first, and the cell suspension is added after the solution to the container.

In another alternative embodiment the cell suspension and the solution are added simultaneously.

It is clear to a skilled person that additional components can be added to the container at any point and that the cell suspension and/or the solution can be pre-cooled prior to filling.

In a preferred embodiment the solution comprises a hyperosmotic cryoprotectant solution selected from the list consisting of

- DMSO
- Glycerol or other sugar alcohols
- Sugars such as glucose, saccharose, raffinose, trehalose
- Amino acids
- Buffer components
- Anorganic salts
- Anti-oxidative agents
- Small molecule additives

The method of the first aspect is hence advantageous compared to a typical cell formulation process e.g. for cryopreservation as the cryoprotectant and the cells are directly added into the final product container e.g. a vial. Thus the exposure time of the cells to a cryoprotectant prior to freezing is reduced. In addition since the mixing of cryoprotectant and the cells is carried out in the final product container validation that all cells supplied in final product containers have been processed under similar conditions is more straightforward that it is the case if mixing of the cryoprotectant and the cells is carried out in a vessel and that cell/cryopreservative mixture is then used to fill the final product container, since cells which are filled first under those conditions could be e.g. frozen prior to the cells which are filled last. Thus, the cells filled last would have been exposed to DMSO prior to freezing for a longer time than the cells filled first.

Thus, in a preferred embodiment of the method of the first aspect the method relates to an automated aseptic method for formulation of cells comprising

- providing a container with a filling volume in the range of 0,01-50 ml
- filling the container with a cell suspension and a solution, comprising DMSO
- closing the container
- mixing the cell suspension and the solution to generate a final suspension

  wherein the container is the final product container.
  wherein the final suspension is not removed again from the final product container for a time in the range of 10 hours to 48 months, preferably 24 hours to 12 months, even more preferably 48 hours to 6 months.

In a preferred embodiment the solution comprises DMSO. Preferably the DMSO concentration in the solution is in the range of 5v/v% to 25 v/v% more preferably it is in the range of 10 v/v% to 20 v/v%, most preferably it if 10 v/v% or 20 v/v%.

The ratio of cell suspension to solution in the final suspension is in the range of 2:1 to 2:1, preferably it is 1:1.

In a preferred embodiment the acceleration is in the range of 2 $m^3/s^2$ - 5 $m^3/s^2$ . It was surprisingly found that this ensured throughout mixing for both 50% vial filling volume and 100% container filling volume while no droplets were formed during mixing at the top of the vial below the stopper.

In a preferred embodiment the container is held in place by a holder during formulation and filling. It is preferred that said holder is connected to a mover and that the mover carries out the movements required for mixing.

Preferably the holder comprises at least one pole and a gripper wherein the pole has a height in the range of 5 - 20 cm. In one embodiment the holder is made from a material selected from the group consisting of stainless steel, aluminum, PEEK (Polyether ether ketone) or PTFE and is connected to the mover via screws or via adhesion e.g. glueing.

The gripper is preferably designed so that it can hold a container with a diameter in the range of 14 - 49 mm and a height of 33 - 76 mm. It is preferably made from stainless steel, aluminum, PEEK or PTFE. Preferably it is connected to the pole via an adapter.

Moreover, in another preferred embodiment a planar system such as the XBot M3-06H with a suitable stator such as the Flyway S3-A from Planar Motors is used. In this embodiment the stator generates a magnetic field that causes the mover to carry out the mixing motions. In this embodiment the holder is centered with respect to the mover and ensures sufficient distance between the mover and the container it holds to exclude any influence of the magnetic field generated by the stator on cells in the container. Moreover, the holder is designed in such a way that it does not scratch the vial surface.

In a preferred embodiment of the method of the first aspect described herein the mixing is achieved via movement of a mover with the following parameters for the axes X, Y and Z

- 14 N, a frequency of 12 hz and a duration of 3s for X, Y and Z axis if no phase shift is applied OR
- 11 N, a frequency of 12 hz and a duration in the range of 2s to 10s for X, Y and Z if a phase shift of 1,57 is applied
- 10 N, a frequency of 10 hz and duration of 5s for X, Y and Z axis if no phase shift is applied OR
- 25 N, a frequency of 12 hz and a duration of 10 s for the X axis if no movement of the Y and Z axis and no phase shift is applied OR
- 11 N, a frequency of 12 hz and a duration of 10s for the X, Y and Z axis if a phase shift of the Y axis of 1,3 is employed OR
- 10 N a frequency of 12 hz and a duration of 10s for the X, Y and Z phase shift of the Y axis of 1,57 is employed.

It was surprisingly found that for both 50% container filling volume and 100% container filling volume these parameters ensured that no drops formed during container handling and particularly during mixing at the container top. Thereby it was prevented that cells in the drops experience different environmental conditions than those in the remaining suspension such as contact to the stopper material or different temperature conditions during subsequent cryopreservation hence ensuring a consisting product quality. In addition, the method ensured adequate mixing i.e. overall product quality and consistency.

To a skilled person it is clear, that reaching the final formulation without droplets being formed on the inside of the container closure depends on the interplay between force (N), frequency (hz), phase shift (rad) and possibly rotation of the movement of the mover - and thereby the container - as well as the mixing time and the filling volume.

With respect to the density of the combined cell suspension and solution e.g. cryoprotectant it was found that variations within cell numbers usually filled and amount of cryoprotectant usually added did not have an effect on the mixing characteristics i.e. the influence of these parameters on the viscosity of the combined cell suspension and solution was apparently negligible (data not shown).

In a further especially preferred embodiment of the method of the first aspect described herein the container is a vial with a filling volume of 2ml, the mixing is achieved via movement of a mover with the following parameters for the axes X, Y and Z

- 14 N, a frequency of 12 hz and a duration of 3s for X, Y and Z axis if no phase shift is applied OR
- 11 N, a frequency of 12 hz and a duration in the range of 2s to 5s if a and a phase shift of 1,57 is applied

and the volume of the cell suspension and the volume of the solution combined fill 50% of the vials filling volume.

It was found that also for the very small volume of 1ml the given parameters achieved efficient mixing without droplet formation on the inside of the container closure. This was surprising as it was expected that such small volumes would be very difficult to mix.

Preferably a planar system such as the XBot M3-06H with a suitable stator such as the Flyway S3-A from Planar Motors is used.

Hence in another especially preferred embodiment of the method of the first aspect described herein the container is a vial with a filling volume of 1 or 2ml, the mixing is achieved via movement of a mover with the following parameters for the axes X, Y and Z

- 14 N, a frequency of 12 hz and a duration of 3s for X, Y and Z axis if no phase shift is applied OR
- 11 N, a frequency of 12 hz and a duration in the range of 2s to 10s for X, Y and Z if a phase shift of 1,57 is applied
- 10 N, a frequency of 10 hz and duration of 5s for X, Y and Z axis if no phase shift is applied OR
- 25 N, a frequency of 12 hz and a duration of 10 s for the X axis if no movement of the Y and Z axis and no phase shift is applied OR
- 11 N, a frequency of 12 hz and a duration of 10s for the X, Y and Z axis if a phase shift of the Y axis of 1,3 is employed OR

- 10 N a frequency of 12 hz and a duration of 10s for the X, Y and Z phase shift of the Y axis of 1,57 is employed.

using a planar system such as the XBot M3-06H with a suitable stator such as the Flyway S3-A from Planar Motors.

Moreover, in another preferred embodiment a planar system such as the XBot M3-06H with a suitable stator such as the Flyway S3-A from Planar Motors is used and a holder is connected to the mover wherein the holder is centered with respect to the mover and ensures sufficient distance between the mover and the container it holds to exclude any influence of the magnetic field of the stator of the planar system on cells in the container and the holder is designed in such a way that it does not scratch the vial surface.

Hence in an especially preferred embodiment the method of the first aspect relates to an automated aseptic method for formulation of cells comprising

- providing a container with a filling volume in the range of 0,01-50 ml
- filling the container with a cell suspension and a solution
- closing the container
- mixing the cell suspension and the solution to generate a final suspension

wherein the container is a final product container and wherein

- the container is a vial,
- said vial has a filling volume of 2ml,
- the cell suspension is filled to the vial first and the solution is filled to the cell suspension in the vial,
- the solution comprises the hyperosmotic cryoprotectant DMSO at a concentration of 10 v/v% or 20 v/v%
- the ratio of cell suspension to solution in the final suspension is 1:1
- the vial is held in place by a holder during formulation and filling which is connected to a mover and the mixing is achieved via movement of said mover with the following parameters for the axes X, Y and Z: 14 N, a frequency of 12 hz and a duration of 3s for X, Y and Z axis if no phase shift is applied OR 11 N, a frequency of 12 hz and a duration in the range of 2s to 5s if a phase shift of 1,57 is applied
- and the volume of the cell suspension and the volume of the solution combined fill 50% of the vials filling volume and

wherein optionally a planar system such as the XBot M3-06H with a suitable stator such as the Flyway S3-A from Planar Motors is used.

Moreover, in an especially preferred embodiment the method of the first aspect relates to an automated aseptic method for formulation of cells consisting of

- providing a container with a filling volume in the range of 0,01-50 ml
- filling the container with a cell suspension and a solution
- closing the container
- mixing the cell suspension and the solution to generate a final suspension

wherein the container is a final product container and wherein

- the container is a vial,
- said vial has a filling volume of 2ml,
- the cell suspension is filled to the vial first and the solution is filled to the cell suspension in the vial,
- the solution comprises the hyperosmotic cryoprotectant DMSO at a concentration of 10 v/v% or 20 v/v%
- the ratio of cell suspension to solution in the final suspension is 1:1
- and the volume of the cell suspension and the volume of the solution combined fill 50% of the vials filling volume.

Furthermore in an especially preferred embodiment the method of the first aspect relates to an automated aseptic method for formulation of cells consisting of

- providing a container with a filling volume in the range of 0,01-50 ml
- filling the container with a cell suspension and a solution
- closing the container
- mixing the cell suspension and the solution to generate a final suspension

wherein the container is a final product container and wherein

- the container is a vial,
- said vial has a filling volume of 2ml,
- the cell suspension is filled to the vial first and the solution is filled to the cell suspension in the vial,
- the solution comprises the hyperosmotic cryoprotectant DMSO at a concentration of 10 v/v% or 20 v/v%
- the ratio of cell suspension to solution in the final suspension is 1:1
- the volume of the cell suspension and the volume of the solution combined fill 50% of the vials filling volume
- and the acceleration is in the range of 2 $m^3/s^2$ - 5 $m^3/s^2$ .

Moreover, in an especially preferred embodiment the method of the first aspect relates to an automated aseptic method for formulation of cells consisting of

- providing a container with a filling volume in the range of 0,01-50 ml
- filling the container with a cell suspension and a solution
- closing the container
- mixing the cell suspension and the solution to generate a final suspension

wherein the container is a final product container and wherein

- the container is a vial,
- said vial has a filling volume of 2ml,
- the cell suspension is filled to the vial first and the solution is filled to the cell suspension in the vial,
- the solution comprises the hyperosmotic cryoprotectant DMSO at a concentration of 10 v/v% or 20 v/v%
- the ratio of cell suspension to solution in the final suspension is 1:1
- the vial is held in place by a holder during formulation and filling which is connected to a mover and the mixing is achieved via movement of said mover with the following parameters for the axes X, Y and Z: 14 N, a frequency of 12 hz and a duration of 3s for X, Y and Z axis if no phase shift is applied OR 11 N, a frequency of 12 hz and a duration in the range of 2s to 5s if a phase shift of 1,57 is applied
- and the volume of the cell suspension and the volume of the solution combined fill 50% of the vials filling volume and

wherein optionally a planar system - also termed planar shaker herein - such as the XBot M3-06H with a suitable stator such as the Flyway S3-A from Planar Motors or XPlanar (Beckhoff Automation) is used.

Preferably the method of the first aspect described herein represents a unit operation in an aseptic filling line. The aseptic filling line is a system designed for the sterile filling of pharmaceutical products. This line operates under strict aseptic conditions to prevent contamination during the filling process. This machine precisely dispenses the sterile product into pre-sterilized containers (e.g., vials, syringes). After filling, containers are sealed with stoppers, followed by a capping process in a manner that maintains sterility.

In one embodiment of the method of the first aspect the step of filling the container with a cell suspension and a solution is characterized in that the cell suspension is provided in at least one bag that is mixed via paddle mixing while all containers are filled or until all cell suspension has been filled in containers

As used herein the term "paddle mixing" refers to a mixing procedure where compression and release of the compression by a paddle induces a fluid movement in a bag, which leads to a mixing effect of the components in the bag.

This has the advantage that a consistent composition of the cell suspension can be ensured even with a very low filling level of the cell suspension in the bag e.g. at the end of the filling process while at the same time the mixing is gentle enough as not to harm the cells.

As specified above the cell suspension and the solution can be filled into the container sequentially or simultaneously. If the filling occurs sequentially i.e. one after the other the filling is preferably followed by a weight check after each dosing. This 100% weight control guarantees to keep the mixing ratio of cell suspension and solution in a specified range.

In a preferred embodiment the weight check is carried out by a stator of the planar system. In an even more preferred embodiment, the weight check is carried out by a weigh cell, which is located on a stator and which can be approached by the mover, that comprises the holder and the container. It is preferred that the weight check comprises a measurement before, a measurement after filling in the cell suspension and a measurement after filling in the solution wherein all values have to be within a range of +/- 5%.

In this embodiment it is furthermore controlled that each the cell suspension and the solution are provided to the container by the action of one filling pump each.

Method according to claim 1 wherein the step of closing the container consists of aseptic stoppering of the container.

In a preferred embodiment the method of the first aspect described herein also comprises at least one of the steps of

- aseptic stoppering of the container
- aseptic capping of the container
- Container Closure Integrity Testing via methods such as tracer gas detection (Helium leak) or vacuum decay.
- Visual inspection of the container after mixing
- labelling of the container
- freezing of the container

Thus, a preferred embodiment the method of the first aspect described herein the step of closing the container consists of aseptic stoppering of the container.

Hence the method described herein in the first aspect also comprises the step of transferred the final product container comprising the final cell suspension to one or more devices for (visual) inspection, labeling and/or continuously freezing the final suspension in the final product container.

Preferably a continuous freezing is a continuous controlled rate freezing process which is a manufacturing technique used to continuously bring containers e.g. vials into the controlled rate freezer, where they undergo a specifically defined and reproducible temperature profile which is essential for controlling the initial ice crystallization and the ice crystal growth which is critical for the cellular survival.

This has the advantage that the time between formulation and freezing is short and that the residence time distribution in e.g. DMSO containing final suspension before freezing - e.g. the time between adding the solution and start of the continuous freezing program or the time between adding the solution and the final suspension having a temperature of equal or below - 120°C -for any given cell undergoing formulation and subsequent freezing is narrow ensuring a consistent product quality.

Hence this embodiment is advantageous as not only the state of the art step of filing the final solution into the final product container after mixing the cells and the cryopreservative is omitted, but the process is continuous up to freezing of the cell suspension thus rendering the novel method more even more time and cost efficient.

In another preferred embodiment the method of the first aspect is characterized in that the time between adding the solution and start of the continuous freezing program for a given container is in the range of 10 min and 5 hours preferably in the range of 20 min and 1 hour.

In an alternative embodiment the method of the first aspect is characterized in that the time between adding the solution and the final suspension having a temperature of equal or below -120°C (e.g. in the range of -120°C and -140°C) for a given container is in the range of 10 min and 5 hours preferably in the range of 80 min and 2,5 hours.

Preferably the continuous freezing step is carried out using a ASKION C-line biobanking system.

Preferably the continuous freezing step is carried out using a ASKION C-line biobanking system using a temperature ramping comprising the steps of initial cooling, controlled ice crystallization and rapid cooling. If a temperature ramping is used preferably a controlled ice nucleation is applied between initial cooling and controlled ice crystallization by a rapid cooling for a short period, preferably in the range of 30 seconds and 3 minutes.

In a preferred embodiment the ASKION C-line biobanking system is connected to the planar (mixing) system via a robot arm and or a conveyor.

In a preferred embodiment the ASKION C-line biobanking system is further connected to an automated storage system such as Askion HS-200 that allows fully automated transfer of frozen samples to the long term storage and thus prevents material from rewarming to product quality critical temperatures.

As stated above it is preferred that the method of the first aspect described herein represents a unit operation in an aseptic filling line and that following the method of the first aspect described herein the final suspension is frozen.

Hence an envisaged overall process comprising these two aspects could entail the steps of

- Cell thawing
- Cell expansion in 2D or 3D culture
- Cell harvest and dissociation
- Formulation using the method of the first aspect described herein
- Stoppering / Sealing
- Capping
- Container closure integrity testing
- Visual inspection
- Labelling
- Freezing

In case the freezing process is not be carried out continuously it is preferred that the container with the final suspension is placed into a rack e.g. a rack of the second aspect prior to freezing.

In one embodiment the automated aseptic method for formulation of cells described herein in the first aspect is a continuous method. Thus, what is described herein relates also to an automated aseptic method for continuous formulation of cells comprising

- providing a container with a filling volume in the range 0,01-50 ml
- filling the container with a cell suspension and a solution
- closing the container
- mixing the cell suspension and the solution to generate a final suspension

wherein the container is a final product container.

As used herein the term "continuous" refers to the fact that at a given time point after the start of the method described herein in the first aspect there are containers already filled which can be process further, containers that are filled and containers still to be filled. Hence if there are downstream unit operations the container containing the final solution should be subjected to these can start working before all cells are formulated as it would be the case in a batch process.

In a preferred embodiment of the method of the first aspect the step of continuously filling the container with a cell suspension and a solution is characterized in that the cell suspension is provided in at least one bag that is mixed via paddle mixing while containers are filled or until all cell suspension has been filled in containers.

In a second aspect what is described herein refers to a rack for freezing containers especially cell product vials.

In a preferred embodiment of the second aspect said rack is used for freezing cell vials. In this embodiment the rack is characterized in that

- It consists of two square plates with the same length in the range of 85 - 135 mm, with the same depth in the range of 85 - 135 mm, and the same thickness in the range of 1-8 mm that are hold by spacers in a distance in the range of 15 and 60 mm
- Spacers and plates are designed and positioned to only minimally impact the horizontal air flow in the CRF and same time offer enough mechanical stability of the rack itself
- The upper plate is positioned in a height that lies above the fill level of the vials
- The rack holds 4 - 36 vials depending on the vial size and format via holes (diameter 14 - 42 mm) in the upper plate and corresponding recesses in the bottom plate
- The recesses offer an improved stability compared to a flat bottom and allow to remove a middle plate that is used in state of the art racks. Additionally, the 2-plate design simplifies the loading and removal of vials due to less potential contact points of the vial with the plates
- Positioning of vials can be either in a checkered-like order or an alternating pattern, in the first cases this leads to a homogeneous airflow resistance across the loaded rack, to achieve that as well with the alternating pattern half-vial-like baffles are introduced into the nest in a way that the baffles of two neighbored nests form a dummy vial. To a skilled person it is clear that also vials can be left out to achieve the same effect.
- The distance from the outer vials to the edge of the rack is equal to half of the distance from one vial to the other within the rack
- it is made from stainless steel, aluminum, graphite or a polymer or a mixture of polymers
- It is used for the procedure of controlled rate freezing as well as transportation to the long term storage and the long term storage itself

To a person skilled in the art it is clear that with using the rack for long term storage the outer dimensions have to match the typically used cryo-storage boxes and the respective towers in a LN2 tank or a -150 °C ultra deep freezer. At the same time in order to use the space in the controlled rate freezer efficiently a number of racks are placed next to each other (e.g. in a 3x2 pattern) and on a number of levels above each other. Within a level it is guaranteed that all cell vials have the same distance to their neighbor vials to ensure a homogeneous flow of a gas phase of liquid nitrogen towards all vials.

In a preferred embodiment of the second aspect the rack is made from polymers selected from the group consisting of Nylon based like PA6 or Polypropylen. To a person skilled in the art it is clear that also metals such as aluminum can be employed however, if metal is used the corners of the rack have to be rounded off.

This rack has the advantage that it is possible to use one and the same rack for controlled rate freezing (CRF) and long-term storage. Optionally, the rack can be inserted into common cryopreservation boxes e.g. 2BA 2 Inch Aluminum Box, Biolife Solutions.

The preferred rack dimensions given above are chosen to accommodate AT closed vials in the format 1 mL to 50 mL from Aseptic Technologies or conventional open (ISO) vials in the format 2R to 50R (e.g. from Gerresheimer, West/Daikyo or Mitsubishi) A skilled person readily understands that the preferred dimensions can be altered in order to store different vials.

Hence in one embodiment of the first aspect the automated aseptic method for formulation of cells comprises the following steps:

- providing a container with a filling volume in the range of 0,01-50 ml
- filling the container with a cell suspension and a solution
- closing the container
- mixing the cell suspension and the solution to generate a final suspension

wherein the container is the final product container and wherein the final product container is placed in a rack of the seconds aspect prior to and for freezing.

Especially preferred embodiments are shown in Fig. 1A and Fig. 1B. These racks due to their dimensions and material can be used in a controlled rate freezer (CRF) and for long-term storage of the frozen material. Hence, they are advantageous compared to the commercially racks offered as standard by the CRF manufacturers (e.g. the CryoMed CRF from Thermo Fisher (1.2/2 mL freezing rack - large 4000703) or the 2101 CRF from Custom Biogenics (2 mL Vial Freezing Rack 42812). It should be noted that the dimensions of both named racks are both for lab cryovials not for AT Vials/2R Vials which have a larger diameter and are validated for pharmaceutical products e.g. they possess a pierceable stopper etc. Moreover, the coated wire tube racks available for AT Vials/2R Vials are only validated a temperature range of -40°C to -121°C, but during controlled rate freezing temperatures below - 120°C may be reached. Both racks also termed cryo-nests were designed for 2mL vials from Aseptic Technologies. The nest design could be also adapted to other vial sizes and formats (especially 2R - 50R vials) while keeping the outer dimensions constant.

In another preferred embodiment the rack is designed in such a way that if it is completely filled with vials the distance between the individual vials is bigger than the distances of the outer vials to the end of the rack perpendicular to the main flow direction, This embodiment is advantageous as shown in example 4 as vertical distances higher than that lead to inhomogeneous flow distributions and hence inhomogeneous temperature distribution leading to inhomogeneous freezing and thawing conditions (cf. Example 4). Overall the preferred embodiment ensures a homogenous temperature distribution within the controlled rate freezer and hence a controlled and reproducible freezing of cells in the vials regardless of where in the freezer the cells happen to be.

EXAMPLES

1. Homogenization experiments

To identify suitable settings that allow mixing a cell-containing suspension via a planar system, a placebo suspension was initially used that mimicked the properties of CryoStor 10 cryomedium with regards to viscosity and density as closely as possible. To replicate the cells, colored polystyrene (PS) particles in suspension with a size of 20 micrometers from microParticles GmbH, Berlin (article no. PS-rot-20.0) were used. Since the sedimentation of solids within a suspension depends on the size and density of the particle, the PS particles were sufficient (data not shown). The concentration of the particles was chosen slightly lower than optimally applicable for potential cell therapy products, as too high concentrations colored the suspension too strongly and did not allow for visual evaluation. 1 or 2 mL of the suspension were manually pipetted into 2mL COP vials from Gerresheimer and left to stand for at least 3 hours so that the particles had completely sedimented to the vial bottom. Then the vials were carefully as not to disturb the sedimented particles placed on a holder which was on a mover (XBot M3-06H from Planar Motors). This mover was then placed on a stator (Flyway S3-A from Planar Motors) and the movement was started. Various parameters for the movement of the mover in the X, Y and Z directions were tried. After the mixing process was completed, the vial was visually inspected. For this purpose, an LED lamp (RaLeno PLV S192) was used to detect particle aggregates and streaks in the suspension. Each setting which ensured a visually sufficient mixture was repeated twice ( cf. Table) not all tried settings are shown.

| No. | Axis | N | f [Hz] | Φ [rad] | Duration [s] | Mixing | Droplet |
|-----|------|-----|--------|---------|--------------|--------|---------|
| I | X | 12 | 12 | 1,57 | 5 | + | + |
|   | Y | 12 | 12 | 1,57 | 5 |   |   |
|   | Z | 12 | 12 | 1,57 | 5 |   |   |
| II | X | 16 | 12 | 0 | 3 | + | + |
|   | Y | 16 | 12 | 0 | 3 |   |   |
|   | Z | 16 | 12 | 0 | 3 |   |   |

(continued)

| No. | Axis | N | f [Hz] | Φ [rad] | Duration [s] | Mixing | Droplet |
|-----|------|-----|--------|---------|--------------|--------|---------|
| III | X | 20 | 12 | 0 | 10 | - | - |
| | Y | 0 | 0 | 0 | 10 | | |
| | Z | 0 | 0 | 0 | 10 | | |
| IV | X | 10 | 12 | 0 | 10 | + at 50% | - |
| | Y | 10 | 12 | 0 | 10 | - at 100% | |
| | Z | 10 | 12 | 0 | 10 | | |
| V | X | 15 | 12 | 0 | 10 | + | + |
| | Y | 15 | 12 | 0 | 10 | | |
| | Z | 15 | 12 | 0 | 10 | | |
| VI | X | 10 | 12 | 0 | 10 | - | - |
| | Y | 10 | 12 | 1,3 | 10 | | |
| | Z | 10 | 12 | 0 | 10 | | |
| VII | X | 12 | 12 | 0 | 10 | + | |
| | Y | 12 | 12 | 1,57 | 10 | | |
| | Z | 12 | 12 | 0 | 10 | | |
| | | | | | | | |
| 1 | X | 14 | 12 | 0 | 3 | + | |
| | Y | 14 | 12 | 0 | 3 | | |
| | Z | 14 | 12 | 0 | 3 | | |
| 2 | X | 11 | 12 | 1,57 | 2-10 | + | - |
| | Y | 11 | 12 | 1,57 | 2-10 | | |
| | Z | 11 | 12 | 1,57 | 2-10 | | |
| 3 | X | 10 | 10 | 0 | 5 | + | - |
| | Y | 10 | 10 | 0 | 5 | | |
| | Z | 10 | 10 | 0 | 5 | | |
| 4 | X | 25 | 12 | 0 | 10 | + | - |
| | Y | 0 | 0 | 0 | 10 | | |
| | Z | 0 | 0 | 0 | 10 | | |
| 5 | X | 11 | 12 | 0 | 10 | + | - |
| | Y | 11 | 12 | 1,3 | 10 | | |
| | Z | 11 | 12 | 0 | 10 | | |
| 6 | X | 10 | 12 | 0 | 10 | + | - |
| | Y | 10 | 12 | 1,57 | 10 | | |
| | Z | 10 | 12 | 0 | 10 | | |

In addition it was found that the setting of No. 6 also worked for a 50% vial filling volume of 5ml (data not shown).

It was surprisingly found that the settings of numbers 1-6 allowed for a throughout mixing for both 50% vial filling volume and 100% container filling volume even with at a 50% vial filling volume of only 1ml while no droplets were formed during mixing at the top of the vial below the stopper.

2. Verification of homogenization experiments

In order to verify the optimal mixing parameters found in Example 1 above a 2mL COP vial from Gerresheimer was filled with 1mL of an iPSC cell suspension formulated in CryoStor 10 (CS10) with a concentration of approx. 15 million cells/mL (stock solution). The stock solution was created by resuspending an iPSC cell pellet in CS10. The suspension was mixed thoroughly several times by pipetting up and down using a 1000 μl Eppendorf pipette. The cell concentration and the cell viability of this mixed stock solution was then determined via Nucleocounter NC202 measurement with the pre-defined method "Count & Viability" according to the manufacturer's instructions. This concentration served as a reference for the subsequent mixing study. The vial was then left to stand for 1 hour, causing almost all cells to sedimented as assessed via visual inspection. The vial was then mixed for 3 seconds using the below mixing parameters.

| Axis | A [N/Nm] | f [Hz] | Φ [rad] | Duration [s] | Mixing | Droplet |
|------|----------|--------|---------|--------------|--------|---------|
| X | 11 | 12 | 1,57 | 2-5 | + | - |
| Y | 11 | 12 | 1,57 | 2-5 | + | - |
| Z | 11 | 12 | 1,57 | 2-5 | + | - |

For mixing the planar system XBot M3-06H mover with the Flyway S3-A stator was used. After mixing the vial was examined for homogeneity visually and or via layer /fraction analysis as follows: To assess the mixing efficiency, it was assumed that the vial contained 5 horizontal layers of 200μl each i.e. the first layer was the top layer and so on with the 5th layer being the bottom layer. One sample of 200μl from each layer was taken carefully using 200ul Eppendorf pipette to obtain as representative a sample as possible from each assumed layer. Each of these samples was analyzed for cell concentration and total cell count via Nucleocounter NC202 with the pre-defined method "Count & Viability" according to the manufacturer's instructions. The samples were compared with the stock solution. It could be shown that using the parameters given above

- the cell concentration corresponded to the cell concentration of the stock solution
- the cell concentration was comparable in all samples taken from different layers of the vial
- no droplets/drops/ splashes were observed above the level of the final solution in the vial.

This experiment was repeated with a vial in which the cells of the cell suspension were completely compressed to the bottom of the vial using a centrifuge (100g for 2,5min). In this experiment it was shown that when the same parameters were applied but after a mixing time of 10 seconds the vial was very well mixed too and no droplets had formed.

3. Automated aseptic formulation method

In this example hiPSCs were used at a concentration of 39E6 cells/mL (stock solution). The cells were provided in StemCellbanker with 0% DMSO as a cell suspension in a 15 mL Falcon Tube. In this example first 0,5 ml of the cell suspension was filled with an Flexicon PF7+ dosing pump into a 2mL COP vial from Gerresheimer using a pump tubing diameter of 0,8 mm, a filling needle with an inner diameter of 1,0 mm and a pump speed of 400 rpm (which leads to an approx. flow rate of 1mL/s). The 2mL vial was held in place by a custom-made holder which was mounted on a XBot M3-06H mover. Then 0,5 ml of a StemCellbanker solution with 20% DMSO (custom made) was filled with an Flexicon PF7+ dosing pump into the vial which was already containing the cell suspension using the same pump tubing diameter of 0,8 mm, a filling needle with an inner diameter of 1,0 mm and a pump speed of 400 rpm. In the next step the vial was closed with a 13mm NovaPure ready to use stopper (West, 1358 4023/50 grey) made from Bromobutyl material. As soon as the vial was closed the cell suspension and the solution were mixed with the XBot M3-06H mover which was placed on a Flyway S3-A to generate a final suspension using the following parameters:

| Axis | A [N/Nm] | f [Hz] | Φ [rad] | Duration [s] | Mixing | Droplet |
|------|----------|--------|---------|--------------|--------|---------|
| X | 11 | 12 | 1,57 | 5 | + | - |
| Y | 11 | 12 | 1,57 | 5 | + | - |
| Z | 11 | 12 | 1,57 | 5 | + | - |

The process was carried out with several vials. In addition, the experiment was repeated in reverse order. First, the StemCellbanker solution with 20% DMSO was added to the vial under the same conditions and then the cell suspension was added before the vial was closed and mixed. After mixing, the stopper was removed from a set number of vials by hand and the inside of the stopper was visually inspected for drops. It was observed that there were no drops on the inside of the stopper. Other vials, which had been filled using the same conditions were subjected to controlled rate freezing directly after closing of the vial using a Planer Kryo 750 system. After reaching a final temperature of -100 °C vials were transferred into long term storage at a temperature lower than -- -140 °C. After thawing the cells were analyzed for post thaw viability, level of apoptosis markers and replating efficiency.

In order to be able to compare the automatically filled and formulated cells with manually formulated and filled cells a portion of the cell suspension used for automatic filling was filled and formulated manually. For this, 2,5 mL of the cell suspension in StemCellbanker with 0% DMSO was transferred by a pipette in a 15 mL Falcon tube. Then 2.5 mL of StemCellBanker with 20% DMSO were added dropwise with a pipette and then mixed manually by pipetting up and down. Finally, 5 vials of 1 mL each were filled with the mixed suspension using a 1mL Eppendorf pipette.

It was found that the automated formulated, filled and mixed cells - especially those where the cells were filled first and the cryoprotectant was added to the cells - were comparable to manually handled cells with regards to the post thaw viability, frequency of apoptotic cells and replating efficiency. Moreover, to assess equal cell distribution as a layer/fraction analysis was carried out as described above i.e. one sample of 200μl from each layer was taken carefully using 200ul Eppendorf pipette to obtain as representative a sample as possible from each assumed layer. Each of these samples was analyzed for cell concentration and total cell count via Nucleocounter NC202 with the pre defined method "Aggregate" according to the manufacturers instructions and compared with the stock solution. It could be shown that:

- the cell concentration corresponded ½ cell concentration of the stock solution (considering the 1:1 dilution)
- the cell concentration was comparable in all samples taken from different layers of the vial
- no droplets/drops/splashes were observed above the level of the final solution in the vial.

4. Computational Fluid Dynamics (CFD) of racks and vials

Computational Fluid Dynamics were used to compare the flow velocity profiles of different racks in a gas stream. The settings were as follows: The program Ansys Fluent 2024 R1 was used to simulate the behavior of a racks according to Fig. 1A and Fig. 1B in a flow field, wherein the racks were completely filled either in a 4-4-4-4 pattern or a 4-3-4-3 pattern and were stacked above each other as it would be the case in a freezing device. In addition, the program Ansys Fluent 2024 R1 was also used to simulate the behavior of a racks designed to have wider gaps between the outer end of the rack and the next rack (up- or sideways). Also here it was simulated that the racks were completely filled in in a 4-4-4-4 pattern or a 4-3-4-3 pattern. For all simulations it was assumed that the vials had an upper diameter of 16mm and a height of 33mm. The rational was that the higher the velocity gradients at the vial surface the higher the convective heat transfer was assumed to be. The outcome was assessed in terms of the features $\zeta$ -'dimensionless pressure loss' - Flow Resistance Coefficient,

$$\zeta \overset{\text{def}}{=} \frac{\Delta p}{\varrho/2 \, v^2}$$

and the Reynolds-Number as 'dimensionless velocity'

$$Re \overset{\text{def}}{=} \frac{v \, d \, \varrho}{\eta}$$

It was found that the pressure loss of the system was higher the higher the velocity gradients at the vial surface were. It was shown that the rack designs of Fig. 1 A and Fig. 1B - spacing the racks at a distance of 2mm - were advantageous for both an alternative number of vials (4-3-4-3) and a regular pattern of vials ( 4-4-4-4 ) configuration, since the velocity gradients was higher between the vials and between different racks of this configuration (data not shown) as compared to settings where racks were spaced at a distance of 20 mm with a design permitting more than 10-15 mm space between individual vials.

Moreover, the ratio of the flow resistance coefficients of the completely filled racks of Fig. 1A and 1B i.e. 1A was filled in a 4-3-4-3 pattern and 1B was filled in a 4-4-4-4 configuration were assessed (cf. Fig. 2). It was found that for lower Re numbers, especially up to around Re 200, the ratio of the flow resistance coefficients increased meaning that especially the configuration of Fig. 1A was beneficial. However, it was surprisingly found that at Re>1000, the ratio of the flow resistance coefficients dropped to around 1, meaning the difference between the different rack filling patterns - i.e. in case of Fig. 1 A and Fig. 1B also the rack design - is especially pronounced at Re<1000.

### 5. Generalization of process parameters towards velocity

To identify suitable velocity settings that allow mixing a cell-containing suspension via a planar system, a placebo suspension of example 1 was used. The vials were carefully as not to disturb the sedimented particles placed on a holder which was on a mover (XBot M3-06H from Planar Motors). This mover was then placed on a stator (Flyway S3-A from Planar Motors) and the movement was started. The mover's time dependent position was controlled and the time dependent sinusoidal movement patterns (trajectories) in x, y and z were described by frequency and amplitude, for further evaluation. After the mixing process was completed, the vial was visually inspected. For this purpose, an LED lamp (RaLeno PLV S192) was used to detect particle aggregates and streaks in the suspension. For the translational movement of the x-coordinate this can be for example described as

$s\_x$ (*t*) position in x at time t in s

$s\_(0, x)$ amplitude in x-direction in m

*f* frequency in Hz

$\phi\_x$ phase shift in rad of the x curve

$$s_x(t) = s_{0,x} \sin(2\pi f\, t + \phi_x)$$

Hence, the first and second derivative regarding time of the trajectories, represents the velocity and the acceleration, where the acceleration causes a momentum which leads, with an appropriate setpoint, to a fluid flow for mixing. The velocity and acceleration for the x-coordinate as an example is thus

$$\frac{\partial\, s_x(t)}{\partial t} = 2\pi f\, s_{0,x} \cdot \cos(2\pi f\, t + \phi_x)$$

$$\frac{\partial^2\, s_x(t)}{\partial t^2} = -4\pi\, f^2\, s_{0,x} \sin(2f\pi t + \phi_x)$$

It was surprisingly found that while assessing the "eigenfrequency" for simple oscillating movements Bessel functions did not lead to appropriate set-points especially mixing without droplet formation and excessive splashing averaging the acceleration over the parametric curve and multiplying with the curve length, a threshold for adequate mixing success within 10s was derived, where above a threshold of

$$\bar{a} = \frac{3\, m^3}{s^2},$$

effective mixing occur for a fill volume of 1mL or 2 mL.

$$\bar{a} = \int \sqrt{a_x^2(t) + a_y^2(t) + a_z^2(t)} \cdot \sqrt{s_x'(t)^2 + s_y'(t)^2 + s_y'(t)^2}\, dt \cdot \int \sqrt{s_x'(t)^2 + s_y'(t)^2 + s_y'(t)^2}\, dt$$

Overall it was found that preferably the acceleration is in the range of 2 $m^3/s^2$ - 5 $m^3/s^2$ in order to ensure throughout mixing for both 50% vial filling volume and 100% container filling volume while no droplets were formed during mixing at the top of the vial below the stopper (data not shown).

### 6. Cell viability in DMSO

The effect of a cryoprotectant comprising DMSO on cell viability was assessed in three independent technical runs. In order to do so iPSC cells (Gibco) were expanded, harvested and suspended in a DMSO containing cryoformulation, resulting in ~ 400 mL formulated cell suspension. The bioprocess container with the already formulated cell suspension was connected to a filling line and 1 mL of cell suspension each was filled into final product containers (2R ISO COP vial). Filling of the whole batch took ~3 h. In parallel to the filling, vials were sampled by removing them from the filling line, opening the vial and measuring the viable cell count. Hence early filled vials corresponded to cells with a short exposure to the cryopreservation solution and the later filled vials to cells with longer exposure. A moderate viability loss was detected over the first two hours and a marked increase in viability loss was observed after two hours. Hence, it was found that cell viability was reduced with increased exposure time of the cells to DMSO at room temperature. Similar results were obtained with other pluripotent and differentiated cell types (data not shown) and have been described in the literature.

**Claims**

1. Automated aseptic method for formulation of cells comprising

   - providing a container with a filling volume in the range of 0,01-50 ml
   - filling the container with a cell suspension and a solution
   - closing the container
   - mixing the cell suspension and the solution to generate a final suspension

2. Method according to claim 1 wherein the container is the final product container.

3. Method according to claim 1 wherein final suspension is not removed again from the final product container for a time in the range of 10 hours to 6 months,

4. Method according to claim 1 wherein the method comprises the additional step of cryopreserving the cells in the final product container.

5. Method according to claim 1 wherein the step of closing the container consists of aseptic stoppering of the container.

6. Method according to claim 4 also comprising the step of continuously freezing the final suspension in the final product container.

7. Method according to claim 6, wherein the time between adding the solution and start of the continuous freezing program for a given container is in the range of 10 min and 5 hours preferably in the range of 20 min and 1 hour or wherein the time between adding the solution and the final suspension having a temperature of equal or below -120°C (e.g. in the range of -120°C and -140°C) for a given container is in the range of 10 min and 5 hours preferably in the range of 80 min and 2,5 hours.

8. Method according to claim 2 wherein the acceleration is in the range of 2 $m^3/s^2$ - 5 $m^3/s^2$ .

9. Method according to claim 1 wherein the mixing is achieved via movement of a mover with the following parameters for the axes X, Y and Z

   - 14 N, a frequency of 12 hz and a duration of 3s for X, Y and Z axis if no phase shift is applied OR
   - 11 N, a frequency of 12 hz and a duration in the range of 2s to 10s for X, Y and Z if a phase shift of 1,57 is applied
   - 10 N, a frequency of 10 hz and duration of 5s for X, Y and Z axis if no phase shift is applied OR
   - 25 N, a frequency of 12 hz and a duration of 10 s for the X axis if no movement of the Y and Z axis and no phase shift is applied OR
   - 11 N, a frequency of 12 hz and a duration of 10s for the X, Y and Z axis if a phase shift of the Y axis of 1,3 is employed OR
   - 10 N a frequency of 12 hz and a duration of 10s for the X, Y and Z phase shift of the Y axis of 1,57 is employed.

10. Method according to claim 1, wherein the container is a vial and said vial has a filling volume in the range of 1-5 ml, most preferred of 2ml.

11. Method according to claim 1, wherein the cell suspension is added to the container first and the solution is added after the cell suspension to the container.

12. Method according to claim 1 wherein the method is an automated aseptic method for continuous formulation.

13. Method according to claim 1 wherein

- the container is a vial,
- said vial has a filling volume of 2ml,
- the cell suspension is filled to the vial first and the solution is filled to the cell suspension in the vial,
- the solution comprises the hyperosmotic cryoprotectant DMSO at a concentration of 10 v/v% or 20 v/v%
- the ratio of cell suspension to solution in the final suspension is 1:1
- the vial is held in place by a holder during formulation and filling which is connected to a mover and the mixing is achieved via movement of said mover with the following parameters for the axes X, Y and Z: 14 N, a frequency of 12 hz and a duration of 3s for X, Y and Z axis if no phase shift is applied OR 11 N, a frequency of 12 hz and a duration in the range of 2s to 5s if a phase shift of 1,57 is applied
- and the volume of the cell suspension and the volume of the solution combined fill 50% of the vials filling volume

14. Method according to claim 1 wherein the container is the final product container and wherein the final product container is placed in a rack prior to and for freezing **characterized in that** it consists of two square plates with the same length in the range of 85 - 135 mm, with the same depth in the range of 85 - 135 mm, and the same thickness in the range of 1-8 mm that are hold by spacers in a distance in the range of 15 and 60 mm.

**Fig. 1**

A

B

**Fig. 2**

Europäisches
Patentamt

European
Patent Office

Office européen
des brevets

## EUROPEAN SEARCH REPORT

**Application Number**

EP 25 21 5784

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/368629 A1 (STOREY ADAM [US]) 22 December 2016 (2016-12-22) * paragraph [0021] * * paragraph [0024] - paragraph [0026] * * paragraphs [0035], [0038], [0042], [0044], [0045] * | 1-14 | INV. C12M1/24 A01N1/147 A61K35/00 B01F31/22 B01L7/00 C12M3/06 |
| A | US 2024/278248 A1 (WENG LINDONG [US] ET AL) 22 August 2024 (2024-08-22) * paragraphs [0022], [0023], [0025] * * paragraphs [0029], [0030] * * paragraph [0032] - paragraph [0044] * * paragraphs [0051], [0058], [0085], [0094], [0095] * * figures 2,2A * | 1-14 | C12M1/00 A01N1/125 A01N1/142 |
| A | US 2023/321618 A1 (SIMMAT OLAF [DE] ET AL) 12 October 2023 (2023-10-12) * paragraph [0127] - paragraph [0130] * * paragraphs [0142], [0366] * * figures 1,3 * | 9,14 | |
| A | CN 117 735 042 A (SUZHOU HANCHAO MEDICAL TECH CO LTD) 22 March 2024 (2024-03-22) * the whole document * | 1-14 | **TECHNICAL FIELDS SEARCHED (IPC)** C12M B01L B01F A61K A01N |
| A | CN 110 124 763 A (LIN WEIYANG) 16 August 2019 (2019-08-16) * the whole document * | 1-14 | |
| A | US 2017/020128 A1 (BOUAITA BELKACEM [FR] ET AL) 26 January 2017 (2017-01-26) * paragraph [0011] * * paragraph [0030] - paragraph [0032] * * paragraph [0036] * | 1-14 | |

The present search report has been drawn up for all claims

| Place of search | Date of completion of the search | Examiner |
|---|---|---|
| Berlin | 1 April 2026 | Cubas Alcaraz, Jose |

EPO FORM 1503 03.82 (P4C01)

**EP 4 748 920 A1**

## ANNEX TO THE EUROPEAN SEARCH REPORT
## ON EUROPEAN PATENT APPLICATION NO.

EP 25 21 5784

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2026

| Patent document cited in search report | Publication date | Patent family member(s) | Publication date |
|---|---|---|---|
| US 2016368629 A1 | 22-12-2016 | NONE | |
| US 2024278248 A1 | 22-08-2024 | US 2024278248 A1 | 22-08-2024 |
| | | WO 2023288181 A1 | 19-01-2023 |
| US 2023321618 A1 | 12-10-2023 | CA 3205237 A1 | 23-06-2022 |
| | | CN 116547063 A | 04-08-2023 |
| | | DE 102020133424 A1 | 15-06-2022 |
| | | EP 4259320 A2 | 18-10-2023 |
| | | ES 3057367 T3 | 02-03-2026 |
| | | US 2023321618 A1 | 12-10-2023 |
| | | WO 2022128809 A2 | 23-06-2022 |
| CN 117735042 A | 22-03-2024 | CN 117735042 A | 22-03-2024 |
| | | CN 219792486 U | 03-10-2023 |
| CN 110124763 A | 16-08-2019 | NONE | |
| US 2017020128 A1 | 26-01-2017 | US 2017020128 A1 | 26-01-2017 |
| | | WO 2015151047 A1 | 08-10-2015 |

EPO FORM P0459

For more details about this annex : see Official Journal of the European Patent Office, No. 12/82

20

**REFERENCES CITED IN THE DESCRIPTION**

**Non-patent literature cited in the description**

- *EudraLex*, vol. 4 (1)